# EUROPEAN PATENT SPECIFICATION

(11) **EP 4 257 974 B1**
(45) Date of publication and mention of the grant of the patent: **23.07.2025**
(21) Application number: 20963831.1
(22) Date of filing: 01.12.2020
(51) Int. Cl.: G01N 33/49, G01N 15/14, G01N 15/1429

(54) **SAMPLE ANALYSIS METHOD AND SAMPLE ANALYZER**
PROBENANALYSEVERFAHREN UND PROBENANALYSATOR
PROCÉDÉ D'ANALYSE D'ÉCHANTILLONS ET DISPOSITIF D'ANALYSE D'ÉCHANTILLONS

(43) Date of publication of application: 11.10.2023
(73) Proprietor: Shenzhen Mindray Bio-Medical Electronics Co., Ltd., Shenzhen, Guangdong 518057 (CN)
(72) Inventor: ZHANG, Ziqian, SHENZHEN, Guangdong 518057 (CN); CHEN, Gengwen, SHENZHEN, Guangdong 518057 (CN)
(74) Representative: KIPA AB
(86) International application number: PCT/CN2020/133039
(87) International publication number: WO 2022/115982

(56) References cited:
- EP-A1- 3 136 081
- EP-B1- 0 613 003
- WO-A1-2019/206300
- CN-A- 103 091 286
- CN-A- 106 483 278
- CN-A- 111 602 052
- CN-A- 111 656 188
- CN-A- 111 684 263
- US-A1- 2015 111 216
- US-B2- 10 379 120

## Description

### TECHNICAL FIELD

The disclosure relates to the field of blood testing, and in particular, to a sample analysis method, a sample analyzer, and a computer-readable storage medium.

### BACKGROUND

Malaria, caused by malaria parasites, is one of the most serious diseases endangering human health. Currently, malaria parasites are detected usually by means of microscopic examination of a blood smear, but this method relies heavily on experience of an operator, requires a high level of expertise for the operator, and is time-consuming.

With the development of blood cell analysis technology, a variety of methods, which can quickly detect erythrocytes infected with malaria parasites by using a hematology analyzer, are currently known.

European Patent Application EP 0613003 B1 discloses a method for staining infected erythrocytes with a plurality of fluorescent dyes under a non-hemolytic condition, so as to better discriminate between reticulocytes and infected erythrocytes.

European Patent Application EP 1406088 A2 discloses a method for detecting malaria parasites with a fluorescent dye under a hemolytic condition, which can implement the classification and counting of malaria parasites, but cannot implement the classification and counting of leukocytes at the same time.

U.S. Patent Application US 2006/0223137 discloses a reagent capable of partially lysing cell membranes of erythrocytes infected with malaria parasites, such that the malaria parasites are retained in the erythrocytes, and a fluorescent dye can pass through the cell membranes. However, erythrocytes infected with malaria parasites cannot be accurately detected when there are high values of reticulocytes in a sample.

Chinese Patent Application CN 106483278 B discloses a method for detecting erythrocytes infected with malaria parasites. In this method, a sample to be tested is treated with a specific fluorescent dye of a specific concentration, allowing more accurate detection of erythrocyte infected with malaria parasites than the solution disclosed in U.S. Patent Application US 2006/0223137.

Chinese Patent Application CN 102016573 B discloses a blood analysis apparatus and a blood analysis method that can classify leukocytes in a test sample into 4 types and detect malaria-infected erythrocytes while reducing the burden on users caused by reagent development. However, in this method, although a same hemolytic agent is used, two blood samples need to be provided for different processing, and differential detection of leukocytes and detection of malaria-infected erythrocytes are performed separately in two tests, causing increased test time, blood volume, and costs of the hemolytic agent.

European Patent EP0613003B1 discloses a reagent for staining malaria infected cells and a method for detecting malaria infected cells using the same, wherein the regent is a staining solution comprising at least one first dye of an Auramine analogue, and a detecting method for malaria infected cells using the reagent by which malaria infected cells, in particular malaria infected erythrocytes, can be stained rapidly and specifically. The method includes: treating a test sample with a staining solution which comprises at least one first dye of an Auramine analogue and at least one second dye of a condensed benzene derivative; and optically detecting stained malaria infected cells.

U.S. Patent US10379120 B2 discloses a blood analyzer including: a specimen preparation unit configured to mix a blood sample with a hemolyzing agent which hemolyzes red blood cells and with a fluorescence-labeled antibody reagent which labels a predetermined surface antigen on blood cells, to prepare a first measurement specimen; a flow cell through which the first measurement specimen prepared by the specimen preparation unit is caused to flow; a light source unit configured to emit light to the first measurement specimen flowing in the flow cell; light receivers configured to respectively receive first scattered light, second scattered light, and first fluorescence which are obtained from blood cells in the first measurement specimen as a result of the emission of the light; and a processing unit configured to identify and count lymphocytes in the first measurement specimen by using first scattered light information based on the first scattered light and second scattered light information based on the second scattered light, and configured to identify and count blood cells having thereon the predetermined surface antigen in the first measurement specimen by using first fluorescence information based on the first fluorescence. The specimen preparation unit mixes the blood sample, a diluent, a hemolyzing agent, and a staining solution for staining plasmodium nucleic acid together, to prepare a second measurement specimen to be used in second measurement. The light receivers respectively receive third scattered light and second fluorescence, which are obtained from blood cells in the second measurement specimen as a result of the emission of the light. The processing unit uses the third scattered light information and the second fluorescence information to count malaria-infected red blood cells in the second measurement specimen.

U.S. Patent Application US20150111216A1 relates to a microfluidic microscopy device. The absorbent structure may include two or more dyes configured to stain different components of a cell or different types of cells. For example, the at least one dry dye for a liquid includes a first fluorescing dye for staining DNA and/or RNA and a second fluorescing dye for staining a plasma membrane or cytoplasmic component. The spectrofluorometer can be used to measure and record fluorescence spectra emitted from a fluorescent dye or dyes associated with the liquid sample in the tapered internal chamber. For example, the optical properties of the cellular components of the liquid sample in the tapered internal chamber is used to differentiate white blood cells from red blood cells. For example, the optical properties of the cellular components of the liquid sample in the tapered internal chamber is used to differentiate Plasmodium-infected and -uninfected red blood cells.

WO2019206300A1 discloses a blood detection method for obtaining a platelet count in a blood sample, which is treated with a first reagent and a hemolytic agent for lysing red blood cells in the blood sample into fragments having light scattering characteristics significantly different from those of platelets.

### SUMMARY

One objective of the disclosure is to provide an improved solution for detecting malaria parasites, in which simultaneous detection of leukocyte parameters and infected erythrocyte parameters can be implemented in one single test, especially in the current leukocyte detection channel. This solution can obtain a variety of detection parameters in one single test, save blood volume for detection, and reduce detection costs compared with the prior art.

Another objective of the disclosure is to provide an improved solution for detecting malaria parasites, in which the detection of infected erythrocyte parameters using two fluorescent dyes under a hemolytic condition can be implemented.

The invention is defined by the method of claim 1 and a sample analyzer according to claim 7. Further aspects of the invention are defined in the dependent claims.

### BRIEF DESCRIPTION OF THE DRAWINGS

FIG. 1 is a schematic appearance diagram of a sample analyzer according to an embodiment of the disclosure;
FIG. 2 is a schematic block diagram of an optical detection apparatus according to an embodiment of the disclosure;
FIG. 3 is a schematic block diagram of an optical detection apparatus according to another embodiment of the disclosure;
FIG. 4 is a schematic flowchart of a sample analysis method according to an embodiment of the disclosure;
FIG. 5 is a schematic flowchart of a sample analysis method according to another embodiment of the disclosure;
FIG. 6 is a schematic diagram of emission spectra of two dyes according to an embodiment of the disclosure;
FIG. 7 is a schematic diagram of an emission spectrum and an excitation spectrum of a dye with a large Stokes shift according to an embodiment of the disclosure;
FIG. 8A is a first scattergram of example 1, and FIG. 8B is a second scattergram of example 1;
FIG. 9A is a first scattergram of example 2, and FIG. 9B is a second scattergram of example 2;
FIG. 10A is a first scattergram of example 3, and FIG. 10B is a second scattergram of example 3;
FIG. 11A is a first scattergram of example 4, and FIG. 11B is a second scattergram of example 4;
FIG. 12A is a first scattergram of example 5, and FIG. 12B is a second scattergram of example 5; and
FIG. 13 is a second scattergram of example 6.

### DESCRIPTION OF THE PREFERRED EMBODIMENTS

The embodiments of the disclosure will be clearly and completely described below in conjunction with the accompanying drawings. Apparently, the described embodiments are merely some, rather than all, of the embodiments of the disclosure. Based on the embodiments of the disclosure, all the other embodiments that would have been obtained by those of ordinary skill in the art without any creative efforts shall fall within the scope of protection of the disclosure.

The serial numbers themselves for the components herein, for example, "first" and "second", are merely used to distinguish the described objects, and do not have any sequential or technical meaning. Moreover, as used in the disclosure, "connection" or "coupling", unless otherwise stated, includes both direct and indirect connections (couplings).

The hematology analyzer used in the disclosure implements classification and counting of particles in a blood sample through a flow cytometry technique using a laser scattering method and a fluorescence staining method in combination. The detection principle of the hematology analyzer is as follows: first, a blood sample is aspirated, and the sample is treated with a hemolytic agent and a fluorescent dye, wherein erythrocytes are destroyed and hemolyzed by the hemolytic agent, while leukocytes will not be hemolyzed, but the fluorescent dye can enter nucleus of the leukocytes with the help of the hemolytic agent and then is bound with nucleic acid substances of the nucleus; and then, particles in the sample are passed through a detection aperture irradiated by a laser beam one by one. When the laser beam irradiates the particles, properties (such as volume, staining degree, size and content of cell contents, density of cell nucleus, etc.) of the particles themselves may block or change a direction of the laser beam, thereby generating scattered light at various angles that corresponds to their properties, and the scattered light can be received by signal detectors to obtain relevant information about a structure and composition of the particles. Forward-scattered light (FS) reflects a number and a volume of particles, side-scattered light (SS) reflects a complexity of a cell internal structure (such as intracellular particles or nucleus), and fluorescence (FL) reflects a content of nucleic acid substances in a cell. The optical information can be used to implement classification and counting of the particles in the blood sample.

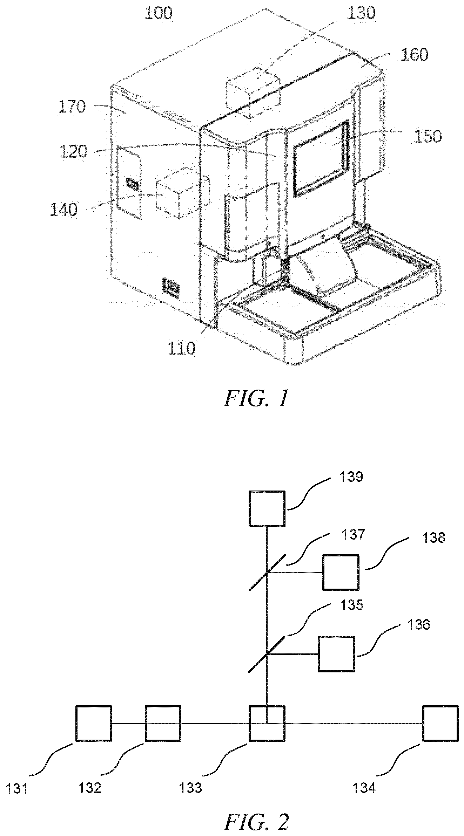

FIG. 1 is a schematic diagram of a hematology analyzer used in an embodiment of the disclosure. The hematology analyzer 100 includes a sampling apparatus 110, a sample preparation apparatus 120, an optical detection apparatus 130, and a processor 140. The hematology analyzer 100 has a liquid circuit system (not shown) for connecting the sampling apparatus 110, the sample preparation apparatus 120 and the optical detection apparatus 130 for liquid transport between these apparatuses.

The sampling apparatus 110 has a pipette with a pipette nozzle and has a driving apparatus for driving the pipette to quantitatively aspirate a blood sample to be tested through the pipette nozzle. The sampling apparatus can transport the aspirated blood sample to be tested to the sample preparation apparatus 120.

The sample preparation apparatus 120 has at least one reaction cell and a reagent supply portion, wherein the at least one reaction cell is configured to receive the blood sample to be tested that is aspirated by the sampling apparatus 110, and the reagent supply portion is configured to supply a hemolytic agent and fluorescent dyes (including a first dye capable of staining leukocytes and a second dye capable of staining infected erythrocytes) to the at least one reaction cell, such that the blood sample to be tested that is aspirated by the sampling apparatus is mixed in the reaction cell with the hemolytic agent and the fluorescent dyes supplied by the reagent supply portion to prepare a test sample solution. The hemolytic agent may be any of existing hemolytic agents used for classification of leukocytes in an automated hematology analyzer. The hemolytic agent may be any one or a combination of a cationic surfactant, a non-ionic surfactant, an anionic surfactant, and an amphiphilic surfactant. Details of the first dye and the second dye will be further explained below.

The optical detection apparatus 130 includes a light source, a flow cell, at least one scattered light detector, and at least two fluorescence detectors, wherein the light source is configured to emit a light beam to irradiate the flow cell; the flow cell is connected with the reaction cell, and particles in the test sample solution are capable of passing through the flow cell one by one; the scattered light detector is configured to detect scattered light signals generated by the particles when passing through the flow cell after being irradiated with the light beam; and the fluorescence detectors are configured to detect fluorescence signals generated by the particles when passing through the flow cell after being irradiated by light.

In some embodiments, the optical detection apparatus 130 includes a forward-scattered light detector for detecting forward-scattered light or a side-scattered light detector for detecting side-scattered light. The optical detection apparatus 130 preferably includes both the forward-scattered light detector and the side-scattered light detector.

FIG. 2 shows a specific example of the optical detection apparatus 130. The optical detection apparatus 130 includes a laser 131, a front optical assembly 132, a flow cell 133, a forward-scattered light detector 134, a first dichroscope 135, a side-scattered light detector 136, a second dichroscope 137, a first fluorescence detector 138, and a second fluorescence detector 139. The first fluorescence detector 138 is configured to detect first fluorescence signals that correspond to the first dye and that are generated by the particles when passing through the flow cell 133 after being irradiated with the light beam, and the second fluorescence detector 139 is configured to detect second fluorescence signals that correspond to the second dye and that are generated by the particles when passing through the flow cell 133 after being irradiated with the light beam. Here, the laser 131, the front optical assembly 132, the flow cell 133, and the forward-scattered light detector 134 are sequentially arranged on an optical axis in a direction of the optical axis, and the front optical assembly is configured such that excitation light emitted by the laser 131 converges in a detection region of the flow cell 133 in a flow direction of the particles, and the particles flowing through the detection region of the flow cell 133 can thus generate scattered light. On one side of the flow cell 133, the first dichroscope 135 is arranged at an angle of 45° to the optical axis. Part of side light generated by the particles when flowing through the detection region of the flow cell 133 is reflected by the first dichroscope 135 and is captured by the side-scattered light detector 136, while the other part of the side light is transmitted through the first dichroscope 135 to the second dichroscope 137, and the second dichroscope 137 is also arranged downstream of the first dichroscope 135 at an angle of 45° to the optical axis. Part of the side light that is transmitted through the first dichroscope 135 is reflected by the second dichroscope 137 and is captured by the first fluorescence detector 138, while the other part of the side light that is transmitted through the second dichroscope 137 is captured by the second fluorescence detector 139.

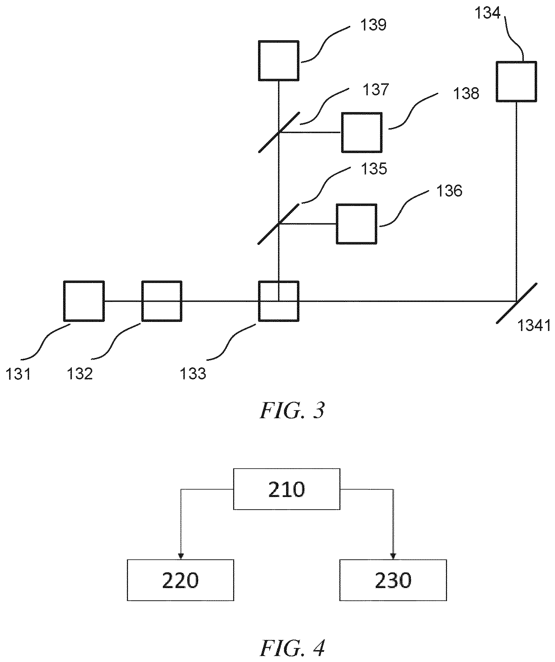

In other embodiments, as shown in FIG. 3, unlike the optical detection apparatus shown in FIG. 2, the forward-scattered light detector 134 may also be arranged to be inclined to the optical axis. On the optical axis, a mirror 1341 is arranged downstream of the flow cell in the direction of the optical axis. The mirror reflects the forward-scattered light of the particles into the forward-scattered light detector 134 arranged to be inclined to the optical axis.

The processor 140 is configured to process optical signals collected by the optical detection apparatus 130, to obtain a required result, for example, may be configured to generate a two-dimensional scattergram or a three-dimensional scattergram based on the collected optical signals, and analyze particles using a gating method on the scattergram. The processor 140 may also be configured to perform visualization processing on an intermediate operation result or a final operation result, and then display same by a display apparatus 150. In embodiments of the disclosure, the processor 140 is configured to implement the method which will be described in detail below. The processor 140 include, but is not limited to, a central processing unit (CPU), a micro controller unit (MCU), a field-programmable gate array (FPGA), a digital signal processor (DSP) and other apparatuses for interpreting computer instructions and processing data in computer software. For example, the processor 140 is configured to execute each computer application program in a computer-readable storage medium, so that the hematology analyzer 100 preforms a corresponding detection process and analyzes, in real time, optical signals detected by the optical detection apparatus 130.

In addition, the hematology analyzer 100 further includes a first housing 160 and a second housing 170. The display apparatus 150 may be, for example, a user interface. The optical detection apparatus 130 and the processor 140 are provided inside the second housing 170. The sample preparation apparatus 120 is provided, for example, inside the first housing 160, and the display apparatus 150 is provided, for example, on an outer surface of the first housing 160 and configured to display test results from the hematology analyzer. In other embodiments, a computer having a display may be remotely and communicatively connected to the hematology analyzer 100. The computer is installed, for example, in a place far away from a laboratory where the hematology analyzer is located, such as in a doctor's consulting room.

Next, the detection method proposed in the disclosure is described in detail. The method proposed in the disclosure and various embodiments thereof are particularly applied to the above hematology analyzer 100, and are particularly implemented by the processor 140 of the above hematology analyzer 100.

In order to implement simultaneous detection of infected erythrocytes and leukocytes in one single test, the disclosure first proposes treating a same blood sample with at least two fluorescent dyes under a hemolytic condition and detecting the treated blood sample, and then identifying both leukocytes and infected erythrocytes based on optical signals obtained in the same test of the same treated blood sample. In the disclosure, one dye is capable of staining leukocytes, while the other dye is capable of staining infected erythrocytes.

FIG. 4 is a schematic flowchart of a sample analysis method 200 according to an embodiment of the disclosure. The sample analysis method 200 includes the following steps.

In step S210, optical signals generated by particles in one test sample solution after being irradiated by excitation light when the particles pass through an optical detection region of an optical detection apparatus one by one are obtained in one single test. In this step, the test sample solution is obtained by treating a blood sample with a hemolytic agent, a first dye and a second dye, the first dye being capable of staining leukocytes, and the second dye being capable of staining infected erythrocytes, wherein the optical signals include scattered light signals, first fluorescence signals corresponding to the first dye, and second fluorescence signals corresponding to the second dye.

Specifically, a blood sample of a subject is first provided, which is generally stored in a test tube, and the sampling apparatus 110 aspirates a portion of the blood sample in the test tube through a pipette and then delivers same to the sample preparation apparatus 120. The portion of the blood sample is mixed with the hemolytic agent, the first dye, and the second dye in the reaction cell of the sample preparation apparatus 120 and incubated for a period of time, such as for 10 to 30s, to ensure that erythrocytes membranes are destroyed by the hemolytic agent and cells are stained, so as to form a test sample solution. The test sample solution is transported to the flow cell 133 of the optical detection apparatus 130 through a liquid circuit system, and particles in the test sample solution are passed through a detection aperture of the flow cell one by one. Then, the scattered light detectors 134 and 136, the first fluorescence detector 138, and the second fluorescence detector 139 respectively detect the scattered light signals, the first fluorescence signals, and the second fluorescence signals generated by the particles when passing through the flow cell after being irradiated by light.

In step S210, the hemolytic agent, the first dye, and the second dye may be added to the blood sample sequentially or simultaneously. It is also possible that the first dye and the second dye are mixed and then added to the blood sample.

In step S220, optical information of leukocytes of the blood sample is obtained based on the first fluorescence signals and at least one type of the scattered light signals. Here, the optical information of leukocytes is optical information related to leukocytes.

For example, the optical information of leukocytes may be a first scattergram. In this step, a first scattergram of the blood sample is generated based on the first fluorescence signals and at least one type of the scattered light signals, and then leukocytes in the test sample solution are classified and/or counted based on the first scattergram. The first scattergram may be a two-dimensional scattergram generated based on forward-scattered light signals and the first fluorescence signals, or a two-dimensional scattergram generated based on side-scattered light signals and the first fluorescence signals, or preferably a three-dimensional scattergram generated based on the forward-scattered light signals, the side-scattered light signals, and the first fluorescence signals. It should be noted that, the scattergram herein is not limited to being presented graphically, and may also be presented in the form of data, such as in the form of digital tables or lists with the same or similar resolution as that of the scattergram, or in any other suitable manner known in the field.

In step S230, optical information of infected erythrocytes of the blood sample is obtained based on the second fluorescence signals and at least one type of the scattered light signals or at least based on the first fluorescence signals and the second fluorescence signals, that is, the optical information of infected erythrocytes is obtained based on the second fluorescence signals, and one type of other optical signals than the second fluorescence signals. Here, the optical information of infected erythrocytes is optical information related to infected erythrocytes.

Similarly, the optical information of infected erythrocytes may be a second scattergram. For example, the second scattergram may be a two-dimensional scattergram generated based on the forward-scattered light signals and the second fluorescence signals or based on the side-scattered light signals and the second fluorescence signals, or a two-dimensional scattergram generated based on the first fluorescence signals and the second fluorescence signals.

In some embodiments, the first dye is a non-nucleic acid-specific dye, and the second dye is a deoxyribonucleic acid (DNA)-specific fluorescent dye. The first fluorescence signals are fluorescence emitted after binding the non-nucleic acid-specific dye with leukocytes, and the second fluorescence signals are fluorescence emitted after binding the nucleic acid-specific dye with malaria-infected cells. The nucleic acid dye can specifically stain infected erythrocytes, and since there difference in nucleic acid content of infected erythrocytes of different types and/or at different development stages, the disclosure can also distinguish between infected erythrocytes of different types and/or at different development stages by staining degree of the second dye while counting infected erythrocytes.

Particularly advantageous, in the optical detection apparatus 130 of the disclosure, excitation light at a single wavelength is used to irradiate the test sample solution in the flow cell, that is, the optical signals are generated by the particles in the test sample solution after being irradiated by the excitation light at the single wavelength when the particles pass through the optical detection region of the optical detection apparatus one by one. In other words, the light source 131 of the optical detection apparatus 130 is configured as a laser that emits an excitation light at a single wavelength. In some embodiments, the light source 131 may be a laser that emits blue-green or red light, for example, may be a laser that emits light with a wavelength of 488 or 520 nanometers.

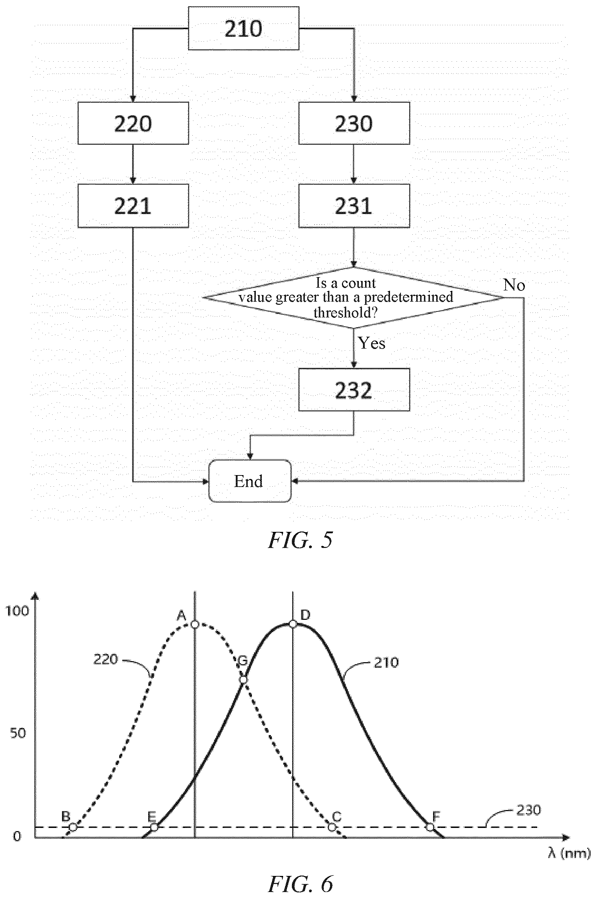

In some embodiments, as shown in FIG. 5, the sample analysis method 200 may further include step 221: classifying and/or counting leukocytes in the test sample solution based on the optical information of leukocytes.

For example, step S221 may include: classifying the leukocytes in the test sample solution into a neutrophil granulocyte population, a lymphocyte population, a monocyte population, and an eosinophil granulocyte population based on the optical information of leukocytes. Specifically, a first scattergram is generated based on the side-scattered light signals and the first fluorescence signals or based on the forward-scattered light signals, the side-scattered light signals and the first fluorescence signals, and on the first scattergram, the leukocytes in the test sample solution are classified into a neutrophil granulocyte population, a lymphocyte population, a monocyte population, and an eosinophil granulocyte population by using a gating technique, and the cell populations are then counted.

In an alternative embodiment, step S221 may include: identifying basophils in the test sample solution and counting the leukocytes in the test sample solution based on the optical information of leukocytes. Specifically, a first scattergram is generated based on the forward-scattered light signals and the first fluorescence signals, and basophils in the test sample solution are identified and the leukocytes in the test sample solution are counted based on the first scattergram. Further, in this embodiment, nucleated erythrocytes in the test sample solution can also be identified while identifying the basophils.

In some embodiments, the sample analysis method 200 may further include identifying immature leukocytes in the test sample solution based on the first fluorescence signals and at least one type of the scattered light signals.

In some embodiments, as shown in FIG. 5, the sample analysis method 200 may further include steps 231 and 232. In step 231, infected erythrocytes are counted based on the optical information of infected erythrocytes, to obtain a count value. For example, a second scattergram is generated based on the forward-scattered light signals and the second fluorescence signals or based on the first fluorescence signals and the second fluorescence signals, and a region representing infected erythrocytes is obtained based on the second scattergram by using a gating technique, and scatters falling into the region are counted to obtain the count value of the infected erythrocytes. In step 232, if the count value of the infected erythrocytes is greater than a predetermined threshold, an alarm prompt is outputted (determining that the blood sample is a malaria positive sample). Further, infected erythrocytes of different types and/or infected erythrocytes at different development stages can also be classified and counted based on the optical information of infected erythrocytes, for example, the infected erythrocytes are classified at least into rings, and for example, the infected erythrocytes can be classified into rings, trophozoites, and schizonts.

Preferably, in order to be able to more accurately distinguish between the leukocytes and the infected erythrocytes by two dyes under the hemolytic condition, particularly when the same excitation light source is used, the first dye and the second dye are selected such that an absolute value of a difference between wavelengths corresponding to peaks of emission spectra of the first dye and the second dye is greater than 30 nanometers and less than 80 nanometers. Alternatively or additionally, the first dye and the second dye are selected such that an overlap between emission spectra of the first dye and the second dye is not greater than 50%. Through such selection of the first dye and the second dye, not only can interference between detecting the first fluorescence signals and detecting the second fluorescence signals be greatly reduced, that is, the degree of discrimination between the first fluorescence signals and the second fluorescence signals is greatly reduced, but the volume and complexity of the optical detection apparatus will not be increased.

FIG. 6 is a schematic diagram of emission spectra of the first dye and the second dye, in which a curve shown by a solid line is an emission spectrum 210 of the first dye, and a curve shown by a dotted line is an emission spectrum 220 of the second dye. A peak point of the emission spectrum 210 of the first dye is D, and a peak point of the emission spectrum 220 of the second dye is A. Here, a difference between respective abscissas of the peak point D and the peak point A (i.e., a difference between wavelengths corresponding to the peaks) is greater than 30 nanometers and less than 80 nanometers. In addition, an overlap between the emission spectrum 210 of the first dye and the emission spectrum 220 of the second dye may be a ratio of the area of a first polygon to the area of a second polygon, where the area of the first polygon is equal to the area of a curved polygon surrounded by three points, namely the point E, the point G, and the point C, and the area of the second polygon is equal to the area of a curved polygon surrounded by the emission spectrum 210 of the first dye (or the emission spectrum 220 of the second dye) and a reference line 230. The reference line 230 is a dotted horizontal line parallel to a horizontal axis as shown in FIG. 6, and the dotted horizontal line is at 5% of a normalized peak of the emission spectrum 210 of the first dye and the emission spectrum 220 of the second dye. The point E and the point F are respectively a left intersection and a right intersection of the emission spectrum 210 of the first dye and the reference line 230, and the point B and the point C are respectively a left intersection and a right intersection of the emission spectrum 220 of the second dye and the reference line 230. Here, the overlap between the emission spectrum 210 of the first dye and the emission spectrum 220 of the second dye is not greater than 50%.

Further, advantageously, especially when irradiated by a single light source, an absolute value of a difference between wavelengths corresponding to the peaks of the emission spectra of the first dye and the second dye is greater than 40 nanometers and less than 80 nanometers, preferably greater than 50 nanometers and less than 80 nanometers, more preferably greater than 50 nanometers and less than 70 nanometers. In this case, the interference between detecting the first fluorescence signals and detecting the second fluorescence signals can be further reduced without increasing the volume and complexity of the optical detection apparatus.

In addition, advantageously, the overlap between the emission spectra of the first dye and the second dye is not greater than 35%, preferably not greater than 15%. In this case, the interference between detecting the first fluorescence signal and detecting the second fluorescence signal can also be further reduced.

In some embodiments, at least one of the first dye and the second dye, particularly the first dye, may be a dye with a large Stokes shift. Here, the dye with a large Stokes shift is a dye with a difference between wavelengths corresponding to respective peaks of an emission spectrum and an excitation spectrum being greater than a predetermined threshold.

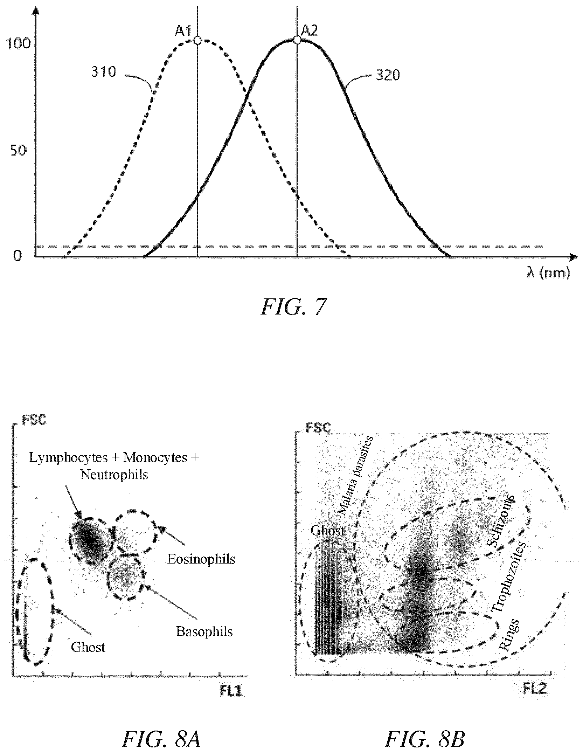

FIG. 7 is a schematic diagram of a dye with a large Stokes shift, in which an excitation spectrum (also referred to as absorption spectrum) 310 of the dye with a large Stokes shift is shown by a dotted line, and an emission spectrum 320 of the dye with a large Stokes shift is shown by a solid line. A peak point of the excitation spectrum 310 is A1, and a peak point of the emission spectrum 320 is A2. A difference between respective abscissas of the peak point A2 and the peak point A1 (i.e., a difference between wavelengths corresponding to respective peaks of the emission spectrum and the excitation spectrum) is greater than a predetermined threshold. The predetermined threshold may be, for example, greater than 30 nanometers and less than 150 nanometers, preferably greater than 50 nanometers and less than 100 nanometers.

By using at least one dye with a large Stokes shift, interference between detecting the first fluorescence signals and detecting the second fluorescence signals can be reduced.

In some embodiments, a parent of the first dye may be a meso-amino-substituted cyanine dye, or a dye parent with a typical electronic push-pull system, such as carbazole and coumarin. For example, the first dye may have a parent structure of general formula I: 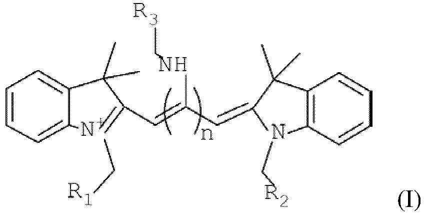 where R1, R2, and R3 are substituents, which can be any element, such as hydrogen element.

For more details of the first dye and the second dye of the disclosure, reference may be made to Chinese patent application no 202011008754.3.

In addition, the disclosure further provides a computer-readable storage medium having instructions stored thereon, wherein the instructions, when executed by a processor, cause the processor to implement the above sample analysis method 200 and one of the embodiments thereof.

The foregoing computer-readable storage medium may be a volatile memory or a non-volatile memory, or may include both a volatile memory and a non-volatile memory. The non-volatile memory may be a read-only memory, a programmable read-only memory, an erasable programmable read-only memory, an electrically erasable programmable read-only memory, a magnetic random access memory, a flash memory, a magnetic surface memory, an optical disc, or a compact disc read-only memory. The magnetic surface memory may be a disk memory or a magnetic tape memory. The volatile memory may be a random access memory, and is used as an external cache. In addition, many forms of RAMs can be applied to the disclosure, such as a static random access memory, a synchronous static random access memory, a dynamic random access memory, a synchronous dynamic random access memory, a double data rate synchronous dynamic random access memory, an enhanced synchronous dynamic random access memory, a synchlink dynamic random access memory, and a direct rambus dynamic random access memory.

Next, the specific embodiments of the disclosure and corresponding results are described by means of the following specific examples.

### Example 1

### Formula of staining reagent:

| | |
|---|---|
| First dye | 50mg |
| Second dye | 50mg |
| Ethylene glycol | 1000g |

The first dye has the following general formula: 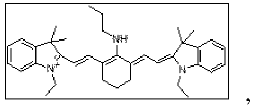 and the second dye has the following general formula:

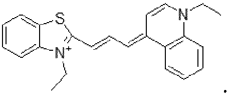

BC-6800 with 68LN hemolytic agent from Mindray Bio-medical Electronics Co., Ltd was used.

Test method: 20 microliters of blood sample and 20 microliters of staining reagent were taken, simultaneously added to 1 ml of hemolytic agent, and incubated for 30 seconds, and after incubation was completed, a flow cytometer was used to detect the sample to be tested to collect forward-scattered light signals, first fluorescence signals, and second fluorescence signals. A first scattergram as shown in FIG. 8A was generated based on the forward-scattered light signals and the first fluorescence signals, and leukocytes were identified based on the first scattergram and were then counted, particularly basophils in the leukocytes could be identified. A second scattergram as shown in FIG. 8B was generated based on the forward-scattered light signals and the second fluorescence signals, and infected erythrocytes were identified based on the second scattergram, and were then classified into rings, trophozoites, and schizonts.

### Example 2

### Formula of staining reagent:

| | |
|---|---|
| First dye | 50mg |
| Second dye | 50mg |
| Ethylene glycol | 1000g |

The first dye has the following general formula: 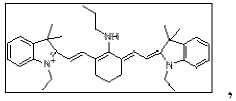 and the second dye has the following general formula:

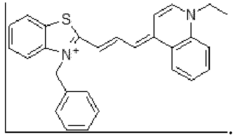

BC-6800 with 68LN hemolytic agent from Mindray Bio-medical Electronics Co., Ltd was used.

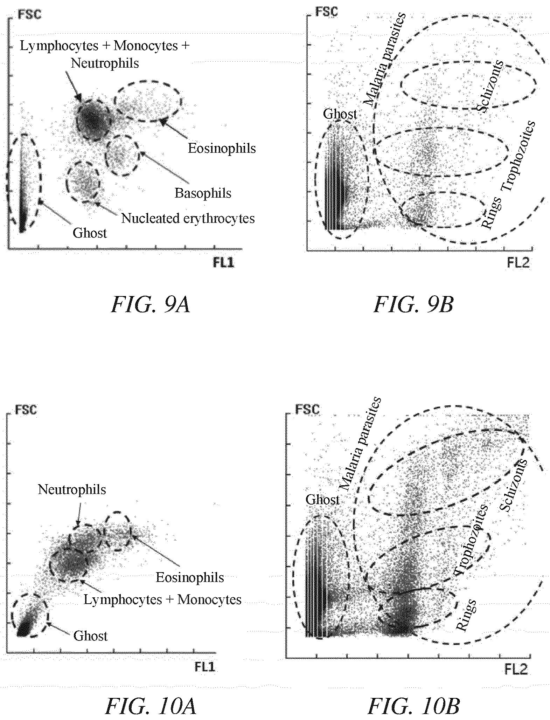

Test method: 20 microliters of blood sample and 20 microliters of staining reagent were taken, simultaneously added to 1 ml of hemolytic agent, and incubated for 30 seconds, and after incubation was completed, a flow cytometer was used to detect the sample to be tested to collect forward-scattered light signals, first fluorescence signals, and second fluorescence signals. A first scattergram as shown in FIG. 9A was generated based on the forward-scattered light signals and the first fluorescence signals, and leukocytes were identified based on the first scattergram and were then counted, particularly nucleated erythrocytes and basophils were identified based on the first scattergram. A second scattergram as shown in FIG. 9B was generated based on the forward-scattered light signals and the second fluorescence signals, and infected erythrocytes were identified based on the second scattergram, and were then classified into rings, trophozoites, and schizonts.

### Example 3

### Formula of staining reagent:

| | |
|---|---|
| First dye | 50mg |
| Second dye | 50mg |
| Ethylene glycol | 1000g |

The first dye has the following general formula: 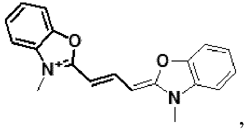 and the second dye has the following general formula:

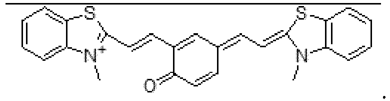

BC-6800 with 68LN hemolytic agent from Mindray Bio-medical Electronics Co., Ltd was used.

Test method: 20 microliters of blood sample and 20 microliters of staining reagent were taken, simultaneously added to 1 ml of hemolytic agent, and incubated for 30 seconds, and after incubation was completed, a flow cytometer was used to detect the sample to be tested to collect forward-scattered light signals, first fluorescence signals, and second fluorescence signals. A first scattergram as shown in FIG. 10A was generated based on the forward-scattered light signals and the first fluorescence signals, and leukocytes were identified based on the first scattergram and were then counted. A second scattergram as shown in FIG. 9B was generated based on the forward-scattered light signals and the second fluorescence signals, and infected erythrocytes were identified based on the second scattergram, and were then classified into rings, trophozoites, and schizonts.

### Example 4

### Formula of staining reagent:

| | |
|---|---|
| First dye | 50mg |
| Second dye | 50mg |
| Ethylene glycol | 1000g |

The first dye has the following general formula: 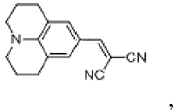 and the second dye has the following general formula:

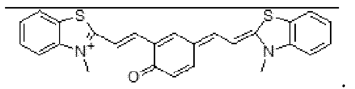

BC-6800 with 68LN hemolytic agent from Mindray Bio-medical Electronics Co., Ltd was used.

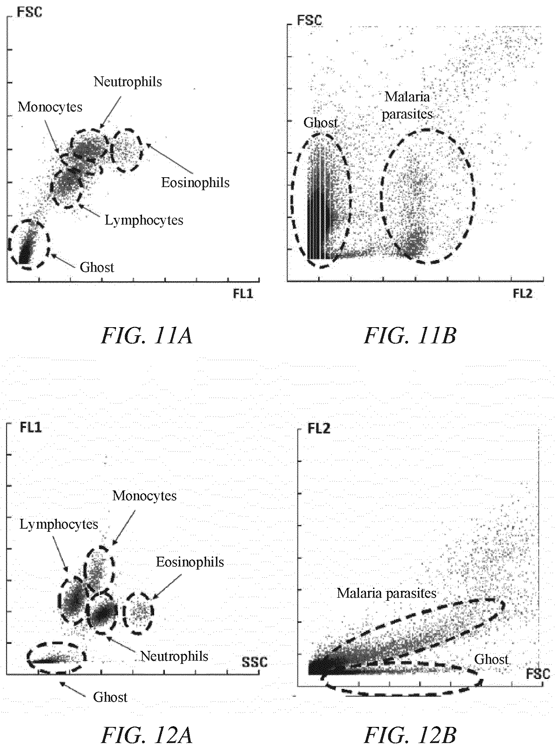

Test method: 20 microliters of blood sample and 20 microliters of staining reagent were taken, simultaneously added to 1 ml of hemolytic agent, and incubated for 30 seconds, and after incubation was completed, a flow cytometer was used to detect a sample to be tested to collect forward-scattered light signals, first fluorescence signals, and second fluorescence signals. A first scattergram as shown in FIG. 11A was generated based on the forward-scattered light signals and the first fluorescence signals, and leukocytes were classified into a neutrophil granulocyte population, a lymphocyte population, a monocyte population, and an eosinophil granulocyte population based on the first scattergram. A second scattergram as shown in FIG. 11B was generated based on the forward-scattered light signals and the second fluorescence signals, and infected erythrocytes were identified based on the second scattergram.

### Example 5

### Formula of staining reagent:

| | |
|---|---|
| First dye | 50mg |
| Second dye | 50mg |
| Ethylene glycol | 1000g |

The first dye has the following general formula: 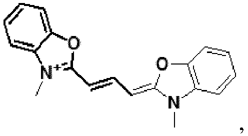 and the second dye has the following general formula:

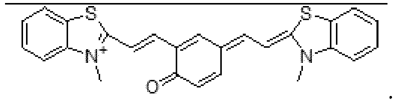

BC-6800 with 68LD hemolytic agent from Mindray Bio-medical Electronics Co., Ltd was used.

Test method: 20 microliters of blood sample and 20 microliters of staining reagent were taken, simultaneously added to 1 ml of hemolytic agent, and incubated for 30 seconds, and after incubation was completed, a flow cytometer was used to detect a sample to be tested to collect forward-scattered light signals, side-scattered light signals, first fluorescence signals, and second fluorescence signals. A first scattergram as shown in FIG. 12A was generated based on the side-scattered light signals and the first fluorescence signals, and leukocytes were classified into a neutrophil granulocyte population, a lymphocyte population, a monocyte population, and an eosinophil granulocyte population based on the first scattergram. A second scattergram as shown in FIG. 12B was generated based on the forward-scattered light signals and the second fluorescence signals, and infected erythrocytes were identified based on the second scattergram.

### Example 6

### Formula of staining reagent:

| | |
|---|---|
| First dye | 50mg |
| Second dye | 50mg |
| Ethylene glycol | 1000g |

The first dye has the following general formula: 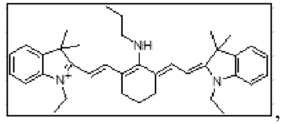 and the second dye has the following general formula:

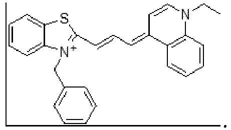

BC-6800 with 68LN hemolytic agent from Mindray Bio-medical Electronics Co., Ltd was used.

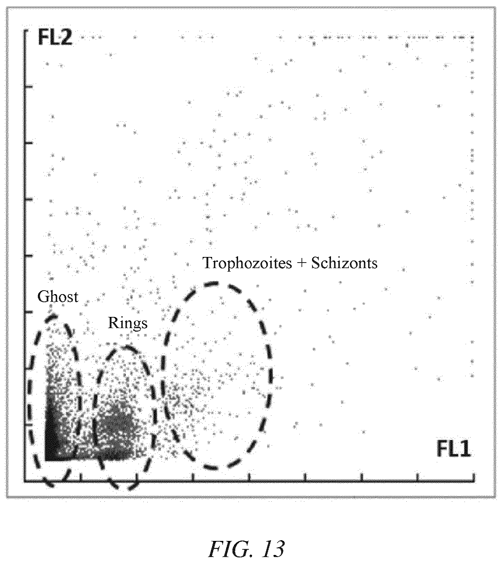

Test method: 20 microliters of blood sample and 20 microliters of staining reagent were taken, simultaneously added to 1 ml of hemolytic agent, and incubated for 30 seconds, and after incubation was completed, a flow cytometer was used to detect a sample to be tested to collect first fluorescence signals and second fluorescence signals. A second scattergram as shown in FIG. 13 was generated based on the first fluorescence signals and the second fluorescence signals, and infected erythrocytes were identified based on the second scattergram, and were then classified into rings, trophozoites, and schizonts.

## Claims

1. A sample analysis method for analyzing a blood sample, comprising:
obtaining (210), in one single test, optical signals generated by particles in a test sample solution after being irradiated by excitation light when the particles pass through an optical detection region of an optical detection apparatus one by one, wherein the test sample solution is obtained by treating the blood sample with a hemolytic agent, a first dye and a second dye, the first dye being capable of staining leukocytes, and the second dye being capable of staining infected erythrocytes, and wherein the optical signals comprise scattered light signals, first fluorescence signals corresponding to the first dye and second fluorescence signals corresponding to the second dye, wherein the first fluorescence signals and the second fluorescence signals are respectively detected by different fluorescence detectors;
obtaining (220) optical information of leukocytes of the blood sample based on the first fluorescence signals and at least one type of the scattered light signals; and
obtaining (230) optical information of infected erythrocytes of the blood sample based on the second fluorescence signals and at least one type of the scattered light signals.

2. The sample analysis method of claim 1, wherein the optical signals are generated by the particles in the test sample solution after being irradiated by the excitation light at a single wavelength when the particles pass through the optical detection region of the optical detection apparatus one by one.

3. The sample analysis method of claim 1 or 2, further comprising:
classifying and/or counting (221) leukocytes in the test sample solution based on the optical information of leukocytes; and/or
identifying nucleated erythrocytes and/or immature leukocytes in the test sample solution based on the first fluorescence signals and at least one type of the scattered light signals.

4. The sample analysis method of claim 3, wherein classifying and/or counting leukocytes in the test sample solution based on the optical information of leukocytes comprises:
classifying the leukocytes in the test sample solution into a neutrophil granulocyte population, a lymphocyte population, a monocyte population and an eosinophil granulocyte population based on the optical information of leukocytes; or
identifying basophils in the test sample solution and counting the leukocytes in the test sample solution based on the optical information of leukocytes.

5. The sample analysis method of any one of claims 1 to 4, further comprising:
counting (231) infected erythrocytes in the test sample solution, and optionally classifying and counting infected erythrocytes of different types and/or infected erythrocytes at different development based on the optical information of infected erythrocytes.

6. The sample analysis method of any one of claims 1 to 5, wherein an absolute value of a difference between wavelengths corresponding to peaks of emission spectra of the first dye and the second dye is greater than 30 nanometers and less than 80 nanometers, and/or an overlap between emission spectra of the first dye and the second dye is not greater than 50%; and/or
wherein a difference between wavelengths corresponding to respective peaks of an emission spectrum and an excitation spectrum of at least one of the first dye and the second dye is greater than a predetermined threshold.

7. A sample analyzer (100), comprising:
a sampling apparatus (110) having a pipette with a pipette nozzle and having a driving apparatus for driving the pipette to quantitatively aspirate a blood sample through the pipette nozzle;
a sample preparation apparatus (120) having at least one reaction cell and a reagent supply portion, wherein the at least one reaction cell is configured to receive the blood sample aspirated by the sampling apparatus, and the reagent supply portion is configured to supply a hemolytic agent, a first dye, and a second dye to the at least one reaction cell, such that the blood sample aspirated by the sampling apparatus is mixed in the reaction cell with the hemolytic agent, the first dye and the second dye supplied by the reagent supply portion, so as to prepare a test sample solution, the first dye being capable of staining leukocytes, and the second dye being capable of staining infected erythrocytes;
an optical detection apparatus (130) comprising a light source (131), a flow cell (133), a scattered light detector (136), a first fluorescence detector (138), and a second fluorescence detector (139), wherein the light source is configured to emit a light beam to irradiate the flow cell, the flow cell is connected with the reaction cell, and particles in the test sample solution are capable of passing through the flow cell one by one, the scattered light detector is configured to detect scattered light signals generated by the particles when passing through the flow cell after being irradiated with the light beam, the first fluorescence detector is configured to detect first fluorescence signals that correspond to the first dye and that are generated by the particles when passing through the flow cell after being irradiated with the light beam, and the second fluorescence detector is configured to detect second fluorescence signals that correspond to the second dye and that are generated by the particles when passing through the flow cell after being irradiated with the light beam; and
a processor (140) configured to perform the following steps: obtaining the scattered light signals, the first fluorescence signals and the second fluorescence signals of the test sample solution in one single test from the optical detection apparatus; obtaining optical information of leukocytes of the blood sample based on the first fluorescence signals and at least one type of the scattered light signals; and obtaining optical information of infected erythrocytes of the blood sample based on the second fluorescence signals and at least one type of the scattered light signals, or obtaining optical information of infected erythrocytes of the blood sample based on the first fluorescence signals and the second fluorescence signals.

8. The sample analyzer (100) of claim 7, wherein the light source (131) is configured to emit an excitation light at a single wavelength.

9. The sample analyzer (100) of claim 7 or 8, wherein the processor (140) is further configured to classify and/or count leukocytes in the test sample solution based on the optical information of leukocytes; and/or
wherein the processor (140) is further configured to identify nucleated erythrocytes and/or immature leukocytes in the test sample solution based on the first fluorescence signals and at least one type of the scattered light signals.

10. The sample analyzer (100) of claim 9, wherein the processor (140) is further configured to, when executing the step of classifying and/or counting leukocytes in the test sample solution based on the optical information of leukocytes:
classify the leukocytes in the test sample solution into a neutrophil granulocyte population, a lymphocyte population, a monocyte population and an eosinophil granulocyte population based on the optical information of leukocytes; or
identify basophils in the test sample solution and count the leukocytes in the test sample solution based on the optical information of leukocytes.

11. The sample analyzer (100) of any one of claims 7 to 10, wherein the processor (140) is further configured to count infected erythrocytes in the test sample solution, and optionally classify and count infected erythrocytes of different types and/or infected erythrocytes at different development stages based on the optical information of infected erythrocytes.

12. The sample analyzer (100) of any one of claims 7 to 11, wherein an absolute value of a difference between wavelengths corresponding to peaks of emission spectra of the first dye and the second dye is greater than 30 nanometers and less than 80 nanometers, and/or an overlap between emission spectra of the first dye and the second dye is not greater than 50%;
wherein a difference between wavelengths corresponding to respective peaks of an emission spectrum and an excitation spectrum of at least one of the first dye and the second dye is greater than a predetermined threshold.

## Patentansprüche

1. Ein Probenanalyseverfahren zur Analyse einer Blutprobe, umfassend:
Das Erhalten (210) von optischen Signalen in einem einzigen Test, die von Partikeln in einer Testprobenlösung erzeugt werden, nachdem diese durch Anregungslicht bestrahlt wurden, wenn die Partikel nacheinander eine optische Detektionsregion einer optischen Detektionsvorrichtung durchqueren, wobei die Testprobenlösung durch Behandlung der Blutprobe mit einem hämolytischen Mittel, einen ersten Farbstoff und einem zweiten Farbstoff erhalten wird, wobei der erste Farbstoff Leukozyten anfärben und der zweite Farbstoff infizierte Erythrozyten anfärben kann, und wobei die optischen Signale Streulichtsignale, erste Fluoreszenzsignale entsprechend dem ersten Farbstoff und zweite Fluoreszenzsignale entsprechend dem zweiten Farbstoff umfassen, wobei die ersten und zweiten Fluoreszenzsignale jeweils durch unterschiedliche Fluoreszenzdetektoren erfasst werden;
das Erhalten (220) von optischen Informationen über Leukozyten der Blutprobe basierend auf den ersten Fluoreszenzsignalen und mindestens einer Art der Streulichtsignale; und
das Erhalten (230) von optischen Informationen über infizierte Erythrozyten der Blutprobe basierend auf den zweiten Fluoreszenzsignalen und mindestens einer Art der Streulichtsignale.

2. Das Probenanalyseverfahren nach Anspruch 1, wobei die optischen Signale von den Partikeln in der Testprobenlösung erzeugt werden, nachdem diese bei einer einzelnen Wellenlänge durch das Anregungslicht bestrahlt wurden, wenn die Partikel nacheinander die optische Detektionsregion der optischen Detektionsvorrichtung durchqueren

3. Das Probenanalyseverfahren nach Anspruch 1 oder 2, ferner umfassen:
Das Klassifizieren und/oder Zählen (221) von Leukozyten in der Testprobenlösung basierend auf den optischen Informationen der Leukozyten; und/oder
Das Identifizieren von kernhaltigen Erythrozyten und/oder unreifen Leukozyten in der Testprobenlösung basierend auf den ersten Fluoreszenzsignalen und mindestens einer Art der Streulichtsignale.

4. Das Proben-Analyseverfahren nach Anspruch 3, wobei das Klassifizieren und/oder Zählen von Leukozyten in der Testprobenlösung basierend auf den optischen Informationen der Leukozyten Folgendes umfasst:
Das Klassifizieren der Leukozyten in der Testprobenlösung in eine Population von neutrophilen Granulozyten, eine Lymphozytenpopulation, eine Monozytenpopulation und eine Population von eosinophilen Granulozyten basierend auf den optischen Informationen der Leukozyten; oder
das Identifizieren von Basophilen in der Testprobenlösung und das Zählen der Leukozyten in der Testprobenlösung basierend auf den optischen Informationen der Leukozyten.

5. Das Probenanalyseverfahren nach einem der Ansprüche 1 bis 4, ferner umfassend:
Das Zählen (231) von infizierten Erythrozyten in der Testprobenlösung, und optional das Klassifizieren und Zählen von infizierten Erythrozyten unterschiedlicher Typen und/oder infizierten Erythrozyten in unterschiedlichen Entwicklungsstadien basierend auf den optischen Informationen der infizierten Erythrozyten.

6. Das Probenanalyseverfahren nach einem der Ansprüche 1 bis 5, wobei ein absoluter Wert der Differenz zwischen Wellenlängen, die den Spitzen der Emissionsspektren des ersten Farbstoffs und des zweiten Farbstoffs entsprechen, größer als 30 Nanometer und kleiner als 80 Nanometer ist, und/oder eine Überlappung zwischen den Emissionsspektren des ersten Farbstoffs und des zweiten Farbstoffs nicht größer als 50 % ist;
und/oder wobei eine Differenz zwischen Wellenlängen, die jeweils den Spitzen eines Emissionsspektrums und eines Anregungsspektrums von mindestens einem der beiden Farbstoffe entsprechen, größer als ein vorbestimmter Schwellenwert ist.

7. Ein Proben-Analysegerät (100), umfassen:
eine Proben-Anahmevorrichtung (110), die eine Pipette mit einer Pipettenspitze und eine Antriebsvorrichtung zum quantitativen Ansaugen einer Blutprobe durch die Pipettenspitze aufweist;
eine Probenvorbereitungsvorrichtung (120) mit mindestens einer Reaktionskammer und einem Reagenzien-Zufuhrbereich, wobei die mindestens eine Reaktionskammer so konfiguriert ist, dass sie die von der Probenahmevorrichtung angesaugte Blutprobe aufnehmen kann, und der Reagenzien-Zufuhrbereich so konfiguriert ist, dass er ein hämolytisches Mittel, einen ersten Farbstoff und einen zweiten Farbstoff an die mindestens eine Reaktionskammer abgibt, sodass die von der Probenahmevorrichtung angesaugte Blutprobe in der Reaktionskammer mit dem vom Reagenzien-Zufuhrbereich zugegebenen hämolytischen Mittel, dem ersten Farbstoff und dem zweiten Farbstoff gemischt wird, um eine Testprobenlösung herzustellen, wobei der erste Farbstoff geeignet ist, Leukozyten anzufärben, und der zweite Farbstoff geeignet ist, infizierte Erythrozyten anzufärben;
eine optische Detektionsvorrichtung (130), umfassend eine Lichtquelle (131), eine Durchflusszelle (133), einen Streulichtdetektor (136), einen ersten Fluoreszenzdetektor (138) und einen zweiten Fluoreszenzdetektor (139), wobei die Lichtquelle so konfiguriert ist, dass sie einen Lichtstrahl zur Bestrahlung der Durchflusszelle aussendet, die Durchflusszelle mit der Reaktionskammer verbunden ist und Partikel in der Testprobenlösung die Durchflusszelle einzeln durchqueren können, der Streulichtdetektor so konfiguriert ist, dass er Streulichtsignale erkennt, die von den Partikeln erzeugt werden, wenn sie nach der Bestrahlung mit dem Lichtstrahl die Durchflusszelle passieren, der erste Fluoreszenzdetektor so konfiguriert ist, dass er erste Fluoreszenzsignale erkennt, die dem ersten Farbstoff entsprechen und von den Partikeln erzeugt werden, wenn sie die Durchflusszelle nach der Bestrahlung mit dem Lichtstrahl passieren, und der zweite Fluoreszenzdetektor so konfiguriert ist, dass er zweite Fluoreszenzsignale erkennt, die dem zweiten Farbstoff entsprechen und von den Partikeln erzeugt werden, wenn sie die Durchflusszelle nach der Bestrahlung mit dem Lichtstrahl passieren; und
einen Prozessor (140), der dazu konfiguriert ist, folgende Schritte auszuführen: Das Erfassen der Streulichtsignale, der ersten Fluoreszenzsignale und der zweiten Fluoreszenzsignale der Testprobenlösung in einem einzigen Test mithilfe der optischen Detektionsvorrichtung; Ermitteln von optischen Informationen über Leukozyten der Blutprobe basierend auf den ersten Fluoreszenzsignalen und mindestens einer Art von Streulichtsignalen; und Ermitteln von optischen Informationen über infizierte Erythrozyten der Blutprobe basierend auf den zweiten Fluoreszenzsignalen und mindestens einer Art von Streulichtsignalen oder Ermitteln von optischen Informationen über infizierte Erythrozyten der Blutprobe basierend auf den ersten und zweiten Fluoreszenzsignalen.

8. Das Proben-Analysegerät (100) nach Anspruch 7, wobei die Lichtquelle (131) so konfiguriert ist, dass sie ein Anregungslicht mit einer einzelnen Wellenlänge emittiert.

9. Das Probenanalysegerät (100) nach Anspruch 7 oder 8, wobei der Prozessor (140) ferner so konfiguriert ist, dass er Leukozyten in der Testprobenlösung basierend auf den optischen Informationen der Leukozyten klassifiziert und/oder zählt;
und/oder wobei der Prozessor (140) ferner so konfiguriert ist, dass er kernhaltige Erythrozyten und/oder unreife Leukozyten in der Testprobenlösung basierend auf den ersten Fluoreszenzsignalen und mindestens einer Art von Streulichtsignalen identifiziert

10. Das Probenanalysegerät (100) nach Anspruch 9, wobei der Prozessor (140) ferner so konfiguriert ist, dass er beim Ausführen des Schritts des Klassifizierens und/oder Zählens von Leukozyten in der Testprobenlösung basierend auf den optischen Informationen der Leukozyten:
Die Leukozyten in der Testprobenlösung in eine Population von neutrophilen Granulozyten, eine Lymphozytenpopulation, eine Monozytenpopulation und eine Population von eosinophilen Granulozyten klassifiziert basierend auf den optischen Informationen der Leukozyten;
oder Basophile in der Testprobenlösung identifiziert und die Leukozyten in der Testprobenlösung basierend auf den optischen Informationen der Leukozyten zählt.

11. Das Probenanalysegerät (100) nach einem der Ansprüche 7 bis 10, wobei der Prozessor (140) ferner so konfiguriert ist, dass er infizierte Erythrozyten in der Testprobenlösung zählt, und optional infizierte Erythrozyten unterschiedlicher Typen und/oder infizierte Erythrozyten in unterschiedlichen Entwicklungsstadien basierend auf den optischen Informationen der infizierten Erythrozyten klassifiziert und zählt.

12. Das Probenanalysegerät (100) nach einem der Ansprüche 7 bis 11, wobei ein absoluter Wert die Differenz zwischen den Wellenlängen, die den Spitzen der Emissionsspektren des ersten Farbstoffs und des zweiten Farbstoffs entsprechen, größer als 30 Nanometer und kleiner als 80 Nanometer ist, und/oder eine Überlappung zwischen den Emissionsspektren des ersten Farbstoffs und des zweiten Farbstoffs nicht größer als 50 % ist;
wobei eine Differenz zwischen den Wellenlängen, die den jeweiligen Spitzen eines Emissionsspektrums und eines Anregungsspektrums von mindestens einem der beiden Farbstoffe entsprechen, größer als ein vorbestimmter Schwellenwert ist.

## Revendications

1. Procédé d'analyse d'échantillons destiné à analyser un échantillon de sang, comprenant les étapes consistant à :
obtenir (210), en un seul test, des signaux optiques générés par des particules dans une solution d'échantillon de test après avoir été irradiées par une lumière d'excitation lorsque les particules traversent une région de détection optique d'un dispositif de détection optique une par une, où la solution d'échantillon de test est obtenue en traitant l'échantillon de sang avec un agent hémolytique, un premier colorant et un second colorant, le premier colorant étant en mesure de colorer des leucocytes, et le second colorant étant en mesure de colorer des érythrocytes infectés, et où les signaux optiques comprennent des signaux lumineux diffractés, des premiers signaux de fluorescence correspondant au premier colorant et des seconds signaux de fluorescence correspondant au second colorant, où les premiers signaux de fluorescence et les seconds signaux de fluorescence sont respectivement détectés par différents détecteurs de fluorescence ;
obtenir (220) des informations optiques de leucocytes de l'échantillon de sang sur la base des premiers signaux de fluorescence et d'au moins un type des signaux lumineux diffractés ; et
obtenir (230) des informations optiques d'érythrocytes infectés de l'échantillon de sang sur la base des seconds signaux de fluorescence et d'au moins un type des signaux lumineux diffractés.

2. Procédé d'analyse d'échantillons selon la revendication 1, dans lequel les signaux optiques sont générés par les particules dans la solution d'échantillon de test après avoir été irradiées par la lumière d'excitation à une seule longueur d'onde lorsque les particules traversent la région de détection optique du dispositif de détection optique une par une.

3. Procédé d'analyse d'échantillons selon la revendication 1 ou 2, comprenant en outre :
le fait de classer et/ou compter (221) des leucocytes dans la solution d'échantillon de test sur la base des informations optiques de leucocytes ; et/ou
le fait d'identifier des érythrocytes nucléés et/ou des leucocytes immatures dans la solution d'échantillon de test sur la base des premiers signaux de fluorescence et d'au moins un type des signaux lumineux diffractés.

4. Procédé d'analyse d'échantillons selon la revendication 3, dans lequel le fait de classer et/ou compter des leucocytes dans la solution d'échantillon de test sur la base des informations optiques de leucocytes comprend :
le fait de classer les leucocytes dans la solution d'échantillon de test dans une population de granulocytes neutrophiles, une population de lymphocytes, une population de monocytes et une population de granulocytes éosinophiles sur la base des informations optiques de leucocytes ; ou
identifier des basophiles dans la solution d'échantillon de test et compter les leucocytes dans la solution d'échantillon de test sur la base des informations optiques de leucocytes.

5. Procédé d'analyse d'échantillons selon l'une quelconque des revendication 1 à 4, comprenant en outre :
le fait de compter (231) des érythrocytes infectés dans la solution d'échantillon de test, et optionnellement de classer et compter des érythrocytes infectés de différents types et/ou des érythrocytes infectés à un développement différent sur la base des informations optiques d'érythrocytes infectés.

6. Procédé d'analyse d'échantillons selon l'une quelconque des revendication 1 à 5, dans lequel une valeur absolue d'une différence entre des longueurs d'onde correspondant à des pics de spectres d'émission du premier colorant et du second colorant est supérieure à 30 nanomètres et inférieure à 80 nanomètres, et/ou un chevauchement entre des spectres d'émission du premier colorant et du second colorant n'est pas supérieur à 50 % ; et/ou
où une différence entre des longueurs d'onde correspondant à des pics respectifs d'un spectre d'émission et d'un spectre d'excitation d'au moins l'un du premier colorant et du second colorant est supérieure à un seuil prédéterminé.

7. Analyseur d'échantillons (100), comprenant :
un dispositif d'échantillonnage (110) présentant une pipette avec une buse de pipette et présentant un dispositif d'entraînement pour entraîner la pipette afin d'aspirer quantitativement un échantillon de sang à travers la buse de pipette ;
un dispositif de préparation d'échantillon (120) présentant au moins une cellule de réaction et une partie de fourniture de réactif, où la au moins une cellule de réaction est configurée pour recevoir l'échantillon de sang aspiré par le dispositif d'échantillonnage, et la partie de fourniture de réactif est configurée pour fournir un agent hémolytique, un premier colorant, et un second colorant à la au moins une cellule de réaction, de telle sorte que l'échantillon de sang aspiré par le dispositif d'échantillonnage est mélangé dans la cellule de réaction avec l'agent hémolytique, le premier colorant et le second colorant fournis par la partie de fourniture de réactif, de sorte à préparer la solution d'échantillon de test, le premier colorant qui est en mesure de colorer des leucocytes, et le second colorant qui est en mesure de colorer des érythrocytes infectés ;
un dispositif de détection optique (130) comprenant une source de lumière (131), une cellule d'écoulement (133), un détecteur de lumière diffractée (136), un premier détecteur de fluorescence (138), et un second détecteur de fluorescence (139), où la source de lumière est configurée pour émettre un faisceau lumineux afin d'irradier la cellule d'écoulement, la cellule d'écoulement est reliée à la cellule de réaction, et les particules dans la solution d'échantillon de test sont en mesure de traverser la cellule d'écoulement une par une, le détecteur de lumière diffractée est configuré pour détecter des signaux lumineux diffractés générés par les particules lorsqu'elles traversent la cellule d'écoulement après avoir été irradiées avec le faisceau lumineux, le premier détecteur de fluorescence est configuré pour détecter des premiers signaux de fluorescence qui correspondent au premier colorant et qui sont générés par les particules lorsqu'elles traversent la cellule d'écoulement après avoir été irradiées avec le faisceau lumineux, et le second détecteur de fluorescence est configuré pour détecter des seconds signaux de fluorescence qui correspondent au second colorant et qui sont générés par les particules lorsqu'elles traversent la cellule d'écoulement après avoir été irradiées avec le faisceau lumineux ; et
un processeur (140) configuré pour réaliser les étapes suivantes : obtenir les signaux lumineux diffractés, les premiers signaux de fluorescence et les seconds signaux de fluorescence de la solution d'échantillon de test en un seul test à partir du dispositif de détection optique ; obtenir des informations optiques de leucocytes de l'échantillon de sang sur la base des premiers signaux de fluorescence et d'au moins un type des signaux lumineux diffractés ; et obtenir des informations optiques d'érythrocytes infectés de l'échantillon de sang sur la base des seconds signaux de fluorescence et d'au moins un type des signaux lumineux diffractés, ou obtenir des informations optiques d'érythrocytes infectés de l'échantillon de sang sur la base des premiers signaux de fluorescence et des seconds signaux de fluorescence.

8. Analyseur d'échantillons (100) selon la revendication 7, dans lequel la source de lumière (131) est configurée pour émettre une lumière d'excitation à une seule longueur d'onde.

9. Analyseur d'échantillons (100) selon la revendication 7 ou 8, dans lequel le processeur (140) est en outre configuré pour classer et/ou compter des leucocytes dans la solution d'échantillon de test sur la base des informations optiques de leucocytes ; et/ou
où le processeur (140) est en outre configuré pour identifier des érythrocytes nucléés et/ou des leucocytes immatures dans la solution d'échantillon de test sur la base des premiers signaux de fluorescence et d'au moins un type des signaux lumineux diffractés.

10. Analyseur d'échantillons (100) selon la revendication 9, dans lequel le processeur (140) est en outre configuré pour, lors de la réalisation de l'étape consistant à classer et/ou compter des leucocytes dans la solution d'échantillon de test sur la base des informations optiques de leucocytes :
classer les leucocytes dans la solution d'échantillon de test dans une population de granulocytes neutrophiles, une population de lymphocytes, une population de monocytes et une population de granulocytes éosinophiles sur la base des informations optiques de leucocytes ; ou
identifier des basophiles dans la solution d'échantillon de test et compter les leucocytes dans la solution d'échantillon de test sur la base des informations optiques de leucocytes.

11. Analyseur d'échantillons (100) selon l'une quelconque des revendications 7 à 10, dans lequel le processeur (140) est en outre configuré pour compter des érythrocytes infectés dans la solution d'échantillon de test, et optionnellement classer et compter des érythrocytes infectés de différents types et/ou des érythrocytes infectés à différents stades de développement sur la base des informations optiques d'érythrocytes infectés.

12. Analyseur d'échantillons (100) selon l'une quelconque des revendications 7 à 11, dans lequel une valeur absolue d'une différence entre des longueurs d'onde correspondant à des pics de spectres d'émission du premier colorant et du second colorant est supérieure à 30 nanomètres et inférieure à 80 nanomètres, et/ou un chevauchement entre des spectres d'émission du premier colorant et du second colorant n'est pas supérieur à 50 % ;
où une différence entre des longueurs d'onde correspondant à des pics respectifs d'un spectre d'émission et d'un spectre d'excitation d'au moins l'un du premier colorant et du second colorant est supérieure à un seuil prédéterminé.
